# EUROPEAN PATENT SPECIFICATION

(11) **EP 3 216 432 B1**
(45) Date of publication and mention of the grant of the patent: **30.12.2020**
(21) Application number: 17159541.6
(22) Date of filing: 07.03.2017
(51) Int. Cl.: D01D 5/34, D01F 1/10, D01F 6/04, D04H 3/14, A61F 13/475, D04H 1/4291, D04H 1/54, D04H 1/541, D04H 1/544, D04H 1/552, D04H 3/007, D04H 3/16

(54) **HIGH BARRIER NONWOVEN FABRIC**
HOCHSPERRENDER VLIESSTOFF
TISSU NON TISSÉ HAUTE PROTECTION

(30) Priority: 09.03.2016 EP 16159433
(43) Date of publication of application: 13.09.2017
(73) Proprietor: Fibertex Personal Care A/S, 9220 Aalborg (DK)
(72) Inventor: Udengaard, Brian, 8520 Lystrup (DK); Hede, Barbara Harling, 9000 Aalborg (DK); Broch, Thomas, 9260 Gistrup (DK)
(74) Representative: Laufhütte, Dieter

(56) References cited:
- EP-A2- 2 283 175
- EP-B1- 2 270 271
- WO-A1-98/50611
- US-A1- 2005 182 198
- US-A1- 2012 088 424
- US-A1- 2012 237 718

## Description

The invention relates to methods for making nonwoven fabrics comprising polymer fibers whose surface properties are modified upon including an additive to the polymer matrix. For example, the hydrophobic and oleophobic character of the fiber surface can be modified for improved barrier properties of the nonwoven, which can be desirable in the hygiene industry.

In the past there were numerous approaches to increase the liquid barrier properties of a nonwoven including, to only name a few, increasing the basis weight of the nonwoven, increasing the content of thinner meltblown fibers in a spunmelt fabric or coating of the fibers.

EP 2 270 271 B1 discloses that adding lipid esters as melt additives to the polymer matrix of the fibers leads to increased barrier properties in a nonwoven fabric. EP 2 778 270 A1 describes that the increased barrier properties may be connected to a specific surface topography of the fibers, which can be obtained by including said melt additives. WO 00/06817 A1 uses in-line hot air knives to increase barrier properties by additive migration in certain regions of a fabric.

The present invention aims to obtain improved results in surface property modification at lower melt additive concentration.

Against this background, the invention relates to a method for making a nonwoven as defined in claim 1.

The invention is based on the theory that fiber surface properties in general and fiber surface structure formation in particular are connected to the additive concentration at the surface. It is further based on the theory that some small molecular additives tend to travel to the surface of the fibers if allowed to migrate within the polymer matrix. The steps proposed by the invention to increase the relative concentration of the additive in the surface region of the fibers correspond to two basic approaches which take account of these theoretical considerations. One of these basic approaches is to increase mobility of the polymer matrix and hence the speed the additive can migrate. The other of these basic approaches is to decrease the distance the additive has to travel to reach the surface by changing the crystallinity.

In one embodiment, bonding at a temperature higher than 40°C below the melting point of the polymer material can be carried out by calandering or hot air through bonding.

According to the invention, the polymer material consists of or comprises a thermoplastic polymer. It may consist of or comprise a polyolefin such as polypropylene and/or polyethylene and/or ethylene-propylene copolymer or a combination of either of these.

Heat treatment after the nonwoven formation procedure means that heat is applied in addition to the heat already applied as per normal production (e.g., during pre-compaction and bonding). It pertains to heat-treatment after the nonwoven has been bonded. This can be achieved by increasing the temperature in the processing of the formed nonwoven fabric (by applying one or several inline heat activation steps) and/or by post-activation when converting the nonwoven into its final application. Generally, heat-treatment after the nonwoven formation procedure refers to heating the nonwoven to temperatures above 100°C, but could also refer to an increased storage temperature of the nonwoven at 30°C, 35°C or 40°C. In some embodiments described in the following, higher temperatures are used. Such heat treatment promotes the mobility of the additive and hence its migration to the surface after the nonwoven fabric has been formed. The heat activation can both directly affect the movement speed of the additive as well as indirectly, by making the polymeric matrix easier to move in for the additive. Heat activation already occurs during in-line thermal bonding, e.g., calander bonding, through-air bonding, ultra sonic bonding and/or radiant heat bonding. According to the invention, additional heat treatments, e.g., application of a drying oven, IR radiation, hot air knives or ultra sonic heating, are applied after the nonwoven formation procedure. They can be in-line and applied to the finished and finally bonded nonwoven, but could also be off-line, or both. It could be heat treatment after the nonwoven has been cut. For example, heat activation after the nonwoven formation procedure can comprise storing the nonwoven at increased temperatures, e.g., at more than 30 or 40°C, for an extended amount of time, e.g., 12 or 24 hours or more. Also, a shorter treatment (e.g., 5 seconds or 0.1 seconds or less) at a temperature of more than 80°C, 100°C, 120°C or 135°C may be suitable employed. The additional heat activation of the invention, in-line and/or off-line, can be in the full web area, but could also be in specific zones only. In one embodiment, heat-treating after the nonwoven fabric formation procedure includes storing the nonwoven at 30°C or more for 12 hours or more or at 100°C or more for 15 minutes or less.

In one embodiment, fillers having a higher thermal conductivity than the polymer material are included to the polymer material. Exemplary fillers include inorganic fillers such as calcium carbonate, which can have a higher thermal conductivity than the polymer matrix (e.g., than polypropylene), allowing faster and more homogeneous transfer of heat within the fiber matrix. This will allow for more benefit from the heat already applied in the processing of the nonwoven and, if any, may increase the effect of heat treatment after the nonwoven formation procedure. The particle size of the filler may be important for the observed effect. In one embodiment, the average particle size of the filler is hence 10 µm or smaller, preferably 1 µm or smaller (ISO 14688). The material may also be chosen to exhibit a thermal conductivity at room temperature of 1 W·m⁻¹·K⁻¹ or greater or more, preferably 2.0 W·m⁻¹·K⁻¹ or more (DIN EN 12664). A preferred thermal conductivity could be 2.7 W·m⁻¹·K⁻¹, which approximately corresponds to that of CaCO₃. In one embodiment, the filler comprises CaCO₃. Suitable CaCO₃ can in one example be either ground CaCO₃ (GCC) or precipitated CaCO₃, or a combination thereof. For example, the CaCO₃ can be micro-CaCO₃ (GCC) having a Plus 325 Mesh of 0.002% and/or mean particle size of 1.6 microns and/or specific surface area of 4.5 m²/g. Such material is, for example, contained in a masterbatch under the trade name "Fiberlink 201S" from A.Schulman. In another example, the CaCO₃ can be nano-CaCO₃ (PCC) having a residue on sieve 45 micron <250 ppm and/or mean particle diameter of 0.07-0.13 microns and/or specific surface area 16 m²/g. Such material is, for example, found under the tradename SOCAL ® U1S2 from Imerys Group.

Step b. is concerned with changing the morphology of the polymeric matrix and hence the distance the melt additive has to navigate when traveling through the matrix.

A nucleating agent increases the number of sites where crystallites begin to form, thereby decreasing the area the crystallites have to grow before they will impinge on each other. Hereby the crystallites will be dimensionally smaller and the additive will have a shorter distance to travel before it reaches the fiber surface. In general melt additives are only able to migrate through the amorphous domains of the polymeric matrix at room temperature, but dependent on the degree of crystallinity (or degree of amorphousness), the geometry and size of the amorphous regions, as well as the conformation and size of the migratory additive, the additive may not be able to migrate effectively at all, as it can be too constricted to move. Therefore, once the processing of the nonwoven is complete, the less constricted the path composed of the amorphous phase, the more additive will be able to reach the surface before the polymer has recrystallized. Suitable nucleating agents can be both inorganic or organic, and insoluble and soluble in the polymer matrix. In one embodiment, the nucleating agent comprises a nonitol, trisamide and/or a sorbitol based nucleating agent. Nonitol can be obtained, for example, from Millad® NX™ 8000 or any masterbatches containing that active component, e.g. NX™ UltraClear GP 110B. Trisamide can be obtained, for example, from Irgaclear XT 386 or any masterbatches containing that active component. A sorbitol based nucleating agent can be obtained, for example, from NC 4 from Mitsui Chemicals.

In one embodiment, an inorganic particulate such as, for example, CaCO₃, the inorganic particulate having a particle size of 10 µm or smaller and preferably 1 µm or smaller can be used as a nucleating agent. It has been found out that inorganic particulate can act as both nucleating agent for influencing polymer crystallinity as well as means for improving heat conduction and hence improving additive mobility. In this respect, it is observed that the improvement of heat conduction appears to be the predominant factor to influence additive migration.

Including branched polymers and/or random co-polymers to the polymer material may result in a polymeric matrix that inherently allows the additive to move more freely and less constricted and therefore faster. Diffusivity may be promoted, e.g., by using/adding branched polymers or random-copolymers as/to the polymer material. If branched polymers and/or random co-polymers are added to the polymer material, the indication of thermal bonding at a temperature higher than 40°C below the melting point of the polymer material refers to the melting point of the mixture.

In one embodiment, multicomponent and in particular bicomponent technology is used and the additive added to only (or predominantly) the polymer feeds eventually forming at least the predominant part of the outermost area of the fibers. The bicomponent could be of core-sheath, but is not limited thereto, and can also be island-in-the-sea, side-by-side, etc. configuration allowing the additive to be added to the surface part of a given bicomponent, e.g. the sheath of the core/sheath bicomponent configuration. Also apart from the additive, the polymeric components of the multicomponent fiber do not have to be identical. In one embodiment, different polymers matrices are employed for the two feeds. The matrix including the additive could also make use of any other measure envisioned in steps a. and b. In one embodiment, the one and the other polymer material both are made of or comprise a thermoplastic polymer, preferably a polyolefin such as polypropylene and/or polyethylene and/or Ethylene-Propylene copolymer or a combination of either of these.

Of course, one or more of the steps and measures mentioned may be employed. For example, heat-treating after the nonwoven fabric formation procedure and including fillers may be used in conjunction. As another example, one or both of the measures for improving the mobility may be used in conjunction with either measure to influence the polymer crystallinity. This has shown to obtain very desirable effects.

Also other steps can be taken in conjunction with the steps and measures mentioned. Among these other steps are spinning thinner fibers, hereby decreasing the distance from the core to the surface. This could be done, but is not limited to, increasing the MFR of the polymer and/or increasing the stress on the fibers during spinning. It is obvious to the one knowledgeable in the state of the art that the migration of a migrating additive is faster/more efficient in a thinner fiber due to the shorter distance to traverse, i.e. the speed will be higher in a fiber with a diameter of 10 micron than in one with a diameter of 20 micron. The stress on fibers during spinning may also be responsible for different extents of stress induced crystallization. Also among these steps are changing the morphology of the polymeric matrix by changing the heating/cooling conditions while processing. As to, for example, the speed of cooling, super cooling is believed to be able to lock the migratory additive in the (amorphous) polymeric matrix. The type of the polymer (homo, co, random, block etc.) and the molecular weight of the polymer may also have an influence on morphology, in addition to the effects rendered with regard to item b.

In one embodiment, the method comprises a continuous process for making a nonwoven fabric, where the fibers are continuously spun and directly dispersed on a carrier belt, preferably comprises making a spunbonded and/or meltblown nonwoven fabric.

In one embodiment, the nonwoven formation procedure includes bonding of the nonwoven fibers. Bonding may, for example, include calendaring and/or air-through bonding. Using a thermal bonding procedure can be advantageous as it already acts as primary heat activation during the nonwoven formation procedure.

According to the invention, the melt additive comprises a fatty acid ester derived from C₈₋₂₅, C₁₀₋₂₀ or C₁₄₋₁₈ saturated or unsaturated carboxylic acids. Examples comprise oleic acid, palmitic acid, myristic acid, myristoleic acid, oleic acid, limoleic acid, linoleic acid and arachidonic acid. The fatty acid ester may be a triglyceride such as, for example, glycerol tristearate.

In one embodiment, the melt additive may be present in an amount of greater 1.2wt% or greater 2wt% based on the total weight of the polymer component. In one embodiment, the melt additive may be present in an amount of smaller 8wt% or smaller 6wt% based on the total weight of the polymer component. For multicomponent fibers, the component containing the additive may in one embodiment comprise higher additive contents. For example, contents of between 4-16wt% are contemplated.

A nonwoven fabric obtainable by the process of the invention comprises polymer fibers where the melt additive is included in the polymer matrix, where the concentration of the melt additive is higher in a surface region than in a core region of the fibers and wherein the fibers comprise a rugged surface topography.

The surface comprises annexes such as fibrils, platelets or flakes that extend outwardly from the surface of the fibers. Fibrils include shapes such as elongate projections and bumps.

The average radial elevation of the annexes from the surface may be in the range of 5 nm - 50 µm, preferably 100 nm - 30 µm and more preferably 500 nm - 20 µm.

Such nonwoven fabric may comprise at least one spunbonded (S) layer which is obtained using the fibers from the additive-comprising melt of the polymer material. It may also comprise at least one meltblown (M) layer which is obtained using the fibers from the additive-comprising melt of the polymer material.

It may also be a spunmelt and preferably a layered SMS-type fabric wherein fibers of at least one of the S and/or M layers are obtained using the fibers from the additive-comprising melt of the polymer material.

Such nonwoven fabric may be used, for example, in a disposable hygiene article such as baby diapers, feminine hygiene products, wipes or adult incontinence products. Within these articles and products, the nonwoven can be utilized for both barrier performance application, e.g. barrier leg cuff in a diaper context, but also converted to act as a masking product, e.g. in a topsheet/core wrap application in feminine hygiene products. Some steps of the present invention such as post production heat activation could be applied to selected zones of benefit to the target application.

Further details and advantages of the invention are hereinafter described with reference to examples.

When reference is made to a hydrophobic melt additive, more specifically PPM17000 High Load Hydrophobic, this refers to a masterbatch with an active component comprising 40wt%.

When reference is made herein to Low Surface Tension Strike Through (LST-ST), it will be determined according to the following method.

The surface tension of a liquid is determined by measuring the force exerted on a platinum Wilhelmy plate at the air-liquid interface. A Kruss tensiometer K11 or equivalent is used. (Available by Kruss USA.) The test is operated in a laboratory environment at 23±2°C and 50±5% relative humidity. The test liquid is placed into the container given by the manufacturer and the surface tension is recorded by the instrument and its software.

The low surface tension fluid strikethrough time test is used to determine the amount of time it takes a specified quantity of a low surface tension fluid, discharged at a prescribed rate, to fully penetrate a sample of a web (and other comparable barrier materials) which is placed on a reference absorbent pad. As a default, this is also called the 32 mN/m Low Surface Tension Fluid Strikethrough Test because of the surface tension of the test fluid and each test is done on two layers of the nonwoven sample simply laid on top of each other.

For the test, the reference absorbent pad is 5 plies of Ahlstrom grade 989 filter paper (10cm x 10cm) and the test fluid is a 32 mN/m low surface tension fluid.

The test is designed to characterize the low surface tension fluid strikethrough performance (in seconds) of webs intended to provide a barrier to low surface tension fluids, such as runny BM, for example.

Lister Strikethrough Tester: The instrumentation is like described in EDANA ERT 153.0-02 section 6 with the following exception: the strike-through plate has a star-shaped orifice of 3 slots angled at 60 degrees with the narrow slots having a 10.0 mm length and a 1.2mm slot width. This equipment is available from Lenzing Instruments (Austria) and from W. Fritz Metzger Corp (USA). The unit needs to be set up such that it does not time out after 100 seconds.

Reference Absorbent Pad: Ahlstrom Grade 989 filter paper, in 10 cm x 10 cm areas, is used. The average strikethrough time is 3.3 + 0.5 seconds for 5 plies of filter paper using the 32 mN/m test fluid and without the web sample. The filter paper may be purchased from Empirical Manufacturing Company, Inc. (USA).

Test Fluid: The 32 mN/m surface tension fluid is prepared with distilled water and 0.42+/-0.001 g/liter Triton-X 100. All fluids are kept at ambient conditions. Electrode-Rinsing Liquid: 0.9% sodium chloride (CAS 7647-14-5) aqueous solution (9g NaCl per 1L of distilled water) is used.

Test Procedure:
- Ensure that the surface tension is 32 mN/m +/- 1 mN/m. Otherwise remake the test fluid.
- Prepare the 0.9% NaCl aqueous electrode rinsing liquid.
- Ensure that the strikethrough target (3.3 +/- 0.5 seconds) for the Reference Absorbent Pad is met by testing 5 plies with the 32 mN/m test fluid as follows:
- Neatly stack 5 plies of the Reference Absorbent Pad onto the base plate of the strikethrough tester.
- Place the strikethrough plate over the 5 plies and ensure that the center of the plate is over the center of the paper. Center this assembly under the dispensing funnel.
- Ensure that the upper assembly of the strikethrough tester is lowered to the pre-set stop point.
- Ensure that the electrodes are connected to the timer.
- Turn the strikethrough tester "on" and zero the timer.
- Using the 5 mL fixed volume pipette and tip, dispense 5 mL of the 32 mN/m test fluid into the funnel.
- Open the magnetic valve of the funnel (by depressing a button on the unit, for example) to discharge the 5 mL of test fluid. The initial flow of the fluid will complete the electrical circuit and start the timer. The timer will stop when the fluid has penetrated into the Reference Absorbent Pad and fallen below the level of the electrodes in the strikethrough plate.
- Record the time indicated on the electronic timer.
- Remove the test assembly and discard the used Reference Absorbent Pad. Rinse the electrodes with the 0.9% NaCl aqueous solution to "prime" them for the next test. Dry the depression above the electrodes and the back of the strikethrough plate, as well as wipe off the dispenser exit orifice and the bottom plate or table surface upon which the filter paper is laid.
- Repeat this test procedure for a minimum of 3 replicates to ensure the strikethrough target of the Reference Absorbent Pad is met. If the target is not met, the Reference Absorbent Pad may be out of spec and should not be used.
- After the Reference Absorbent Pad performance has been verified, nonwoven web samples may be tested.
- Cut the required number of nonwoven web specimens. For web sampled off a roll, cut the samples into 10 cm by 10 cm sized square specimens. For web sampled off of a product, cut the samples into 15 by 15 mm square specimens. The fluid flows onto the nonwoven web specimen from the strike through plate. Touch the nonwoven web specimen only at the edge.
- Neatly stack 5 plies of the Reference Absorbent Pad onto the base plate of the strikethrough tester.
- Place the nonwoven web specimen on top of the 5 plies of filter paper. Two plies of the nonwoven web specimen are used in this test method. If the nonwoven web sample is sided (i.e., has a different layer configuration based on which side is facing in a particular direction), the side facing the wearer (for an absorbent product) faces upwards in the test.
- Place the strikethrough plate over the nonwoven web specimen and ensure that the center of the strikethrough plate is over the center of the nonwoven web specimen. Center this assembly under the dispensing funnel.
- Ensure that the upper assembly of the strikethrough tester is lowered to the pre-set stop point.
- Ensure that the electrodes are connected to the timer. Turn the strikethrough tester "on" and zero the timer.
- Run as described above.
- Repeat this procedure for the required number of nonwoven web specimens. A minimum of 5 specimens of each different nonwoven web sample is required. The average value is the 32 mN/m low surface tension strikethrough time in seconds.

### Examples 1-4:

Spunbonded (S) single layer nonwoven fabrics were produced from 100-x wt% Ziegler-Natta polypropylene and x wt% of a hydrophobic melt additive (PPM17000 High Load Hydrophobic) and thermally bonded. The single S-layers had a weight of 20 g/m². The contents of the hydrophobic additive in Examples 1-4 are summarized in Table 1.

**Table 1:**

| Example | X [wt%] |
|---|---|
| 1 | 0 |
| 2 | 3 |
| 3 | 6 |
| 4 | 10 |

Examples 1-4 were tested for Low Surface Tension Strike Through (LST-ST). The results are summarized in Table 2.

**Table 2: LST-ST**

| Example | 1 | 2 | 3 | 4 |
|---|---|---|---|---|
| | 5.20 | 14.13 | 8.25 | 10.22 |
| | 5.12 | 7.04 | 12.45 | 16.70 |
| | 4.27 | 6.97 | 11.93 | 9.84 |
| | 4.15 | 8.20 | 11.64 | 19.81 |
| | 4.80 | 7.41 | 14.13 | 12.64 |
| | 4.95 | 7.54 | 9.87 | 13.26 |
| | 3.80 | 7.53 | 10.16 | 11.41 |
| | 4.30 | 7.16 | 13.05 | 9.39 |
| | 4.66 | 8.68 | 9.25 | 20.75 |
| | 4.30 | 7.37 | 12.17 | 8.80 |
| | 5.08 | 6.98 | 10.32 | 10.40 |
| | 6.11 | 7.24 | 10.16 | 16.93 |
| | 4.74 | 7.89 | 11.65 | 14.83 |
| | 5.21 | 9.47 | 11.72 | 12.56 |
| | 6.25 | 8.58 | 11.19 | 16.33 |
| Average | 4.86 | 8.15 | 11.20 | 13.59 |
| Std. Dev | 0.68 | 1.81 | 1.53 | 3.81 |
| Min | 3.80 | 6.97 | 8.25 | 8.80 |
| Max | 6.25 | 14.13 | 14.13 | 20.75 |

### Example 5-7:

Three S single layer nonwovens were produced from 100% Ziegler-Natta polypropylene and thermally bonded. The single S-layer had a weight of 20 g/m². After the web making process of the nonwovens they were thermally treated with an in-line Omega Drying oven at 90°C, 120°C and 135°C, for Example 5, 6 and 7, respectively.

### Example 8:

An S single layer nonwoven was produced from 100% Ziegler-Natta polypropylene and thermally bonded. The single S-layer had a weight of 20 g/m². After the web making process the nonwoven was thermally treated with an in-line IR-heater set to 65% power at the center and 60% at the edge of the nonwoven web.

### Example 9:

An S single layer nonwoven was produced from 100% Ziegler-Natta polypropylene and thermally bonded. The single S-layer had a weight of 20 g/m². After the web making process the nonwoven was thermally treated with an in-line Omega Drying oven at 120°C. Opposite Example 6, the through put had been decreased in the production of the material, resulting in a decreased line speed to increase the duration of the heat treatment. The resulting heat treatment of Example 9 was 15 % longer than that of Example 6.

Table 3 below shows the resulting LST-ST measured on Example 5-9.

**Table 3:**

| Example | 5 | 6 | 7 | 8 | 9 |
|---|---|---|---|---|---|
| | 6.93 | 8.15 | 8.41 | 5.73 | 6.45 |
| | 6.98 | 6.56 | 9.00 | 7.45 | 8.82 |
| | 7.36 | 6.97 | 7.21 | 6.57 | 7.59 |
| | 5.88 | 7.30 | 6.63 | 8.21 | 8.31 |
| | 6.30 | 7.54 | 6.01 | 6.84 | 7.40 |
| | 7.57 | 7.39 | 6.41 | 6.62 | 10.42 |
| | 3.27 | 5.95 | 7.18 | 8.17 | 8.14 |
| | 6.30 | 6.31 | 6.83 | 6.46 | 6.99 |
| | 6.15 | 6.99 | 6.56 | 6.23 | 5.97 |
| | 5.96 | 8.25 | 6.03 | 6.60 | 7.97 |
| Average | 6.27 | 7.14 | 7.03 | 6.89 | 7.81 |
| Std. Dev | 1.21 | 0.74 | 0.98 | 0.81 | 1.26 |
| Min | 3.27 | 5.95 | 6.01 | 5.73 | 5.97 |
| Max | 7.57 | 8.25 | 9.00 | 8.21 | 10.42 |

### Examples 10-13:

Four S single layer nonwovens were produced from 90wt% Ziegler-Natta polypropylene and 10wt% of a hydrophobic melt additive (PPM17000 High Load Hydrophobic) and thermally bonded. The single S-layer had a weight of 20 g/m². After the web making process of the nonwovens they were thermally treated with an in-line Omega Drying oven set to 90 °C, 105°C, 120°C and 135°C for Example 10, 11, 12 and 13, respectively.

### Examples 14-17:

Four S single layer nonwovens were produced from 90wt% Ziegler-Natta polypropylene and 10wt% hydrophobic melt additive (PPM17000 High Load Hydrophobic) and thermally bonded. The single S-layer had a weight of 20 g/m2. After the web making process of the nonwovens they were thermally treated with an in-line IR-heater set to 50% power at the center and 45% at the edge of the nonwoven web, 60% power at the center and 55% at the edge of the nonwoven web, 65% power at the center and 50% at the edge of the nonwoven web, and 70% power at the center and 65% at the edge of the nonwoven web for Example 14, 15, 16 and 17, respectively.

### Example 18:

An S single layer nonwoven was produced from 90 wt% Ziegler-Natta polypropylene and 10 wt% hydrophobic melt additive (PPM17000 High Load Hydrophobic) and thermally bonded. The single S-layer had a weight of 20 g/m². After the web making process of the nonwoven it was thermally treated with an in-line IR-heater set to 65% power at the center and 60% at the edge of the nonwoven web, followed by heating in an Omega Drying oven at 120°C.

The hydrophobic additive content and heat treatment for Examples 10-18 are summarized in Table 4 below.

**Table 4:**

| | Configuration | | |
|---|---|---|---|
| | S | | |
| | 20 g/m² | | |
| Example | PPM17000 in S [%] | IR heater, center/edge [%] | Omega Drying oven temperature [°C] |
| 10 | 10 | N/A | 90 |
| 11 | 10 | N/A | 105 |
| 12 | 10 | N/A | 120 |
| 13 | 10 | N/A | 135 |
| 14 | 10 | 50/45 | N/A |
| 15 | 10 | 60/55 | N/A |
| 16 | 10 | 65/60 | N/A |
| 17 | 10 | 70/65 | N/A |
| 18 | 10 | 65/60 | 120 |

LST-ST was measured on Example 10-18. The results are shown in Table 7.

**Table 5:**

| Example | 10 | 11 | 12 | 13 | 14 | 15 | 16 | 17 | 18 |
|---|---|---|---|---|---|---|---|---|---|
| | 76.00 | 101.24 | 234.77 | 395.48 | 15.26 | 14.84 | 104.54 | 98.37 | 215.35 |
| | 61.97 | 49.95 | 276.72 | 657.52 | 17.00 | 12.71 | 152.30 | 111.38 | 110.34 |
| | 46.90 | 112.09 | 146.37 | 430.89 | 20.50 | 14.10 | 112.22 | 110.01 | 198.74 |
| | 74.91 | 88.00 | 273.29 | 474.56 | 19.56 | 28.91 | 266.32 | 98.23 | 410.14 |
| | 42.44 | 109.78 | 58.76 | 198.51 | 15.18 | 18.06 | 95.32 | 126.05 | 217.10 |
| | 79.87 | 142.88 | 305.67 | 494.47 | 22.90 | 19.65 | 156.70 | 55.01 | |
| | 38.29 | 40.85 | 140.77 | 538.19 | 11.38 | 15.53 | 304.52 | 64.02 | |
| | 67.46 | 37.91 | 196.46 | 380.06 | 18.17 | 20.92 | 138.70 | 113.62 | |
| | 35.59 | 93.40 | 51.80 | 609.90 | 24.31 | 25.86 | 301.90 | 70.28 | |
| | 65.22 | 213.77 | 178.43 | 437.63 | 19.12 | 16.57 | 211.70 | 74.74 | |
| | | | | | 20.24 | 16.76 | 270.70 | 52.05 | |
| | | | | | 16.97 | 19.58 | 317.12 | 86.01 | |
| | | | | | 36.15 | 30.45 | 216.29 | 33.97 | |
| | | | | | 12.96 | 30.45 | 273.13 | 76.24 | |
| | | | | | 19.00 | 14.05 | 408.33 | 96.01 | |
| Average | 58.87 | 98.99 | 186.30 | 461.72 | 19.25 | 19.90 | 221.99 | 84.40 | 230.33 |
| Std. Dev | 16.65 | 52.77 | 88.53 | 128.77 | 5.81 | 6.15 | 93.35 | 26.36 | 109.69 |
| Min | 35.59 | 37.91 | 51.80 | 198.51 | 11.38 | 12.71 | 95.32 | 33.97 | 110.34 |
| Max | 79.87 | 213.77 | 305.67 | 657.52 | 36.15 | 30.45 | 408.33 | 126.05 | 410.14 |

### Example 19:

An S single layer nonwoven was produced from 90wt% Ziegler-Natta polypropylene and 10wt% hydrophobic melt additive (PPM17000 High Load Hydrophobic) and thermally bonded. The single S-layer had a weight of 20 g/m². Compared to Example 4, the temperature of the calender thermally bonding the nonwoven was increased with +10°C.

Table 6 below shows the LST-ST results from Example 19.

**Table 6:**

| | LST ST [s] |
|---|---|
| Example | 19 |
| | 19.34 |
| | 14.72 |
| | 20.11 |
| | 14.50 |
| | 60.64 |
| | 15.93 |
| | 27.21 |
| | 18.45 |
| | 32.66 |
| | 46.12 |
| | 36.68 |
| | 16.23 |
| | 17.71 |
| | 26.82 |
| | 41.66 |
| Average | 27.25 |
| Std.dev. | 13.75 |
| Min | 14.50 |
| Max | 60.64 |

It can be see that when increasing the calender temperature with 10 °C, the LST ST increases from 13.59 seconds (4) to 27.25 seconds (19).

### Example 20:

An S single layer nonwoven was produced from 90% Ziegler-Natta polypropylene and 10 wt% of a hydrophobic melt additive (PPM17000 High Load Hydrophobic) and thermally bonded. The single S-layer had a weight of 20 g/m2. After the web making process the nonwoven was thermally treated with an in-line Omega Drying oven at 120°C. As Example 13, the through put had been decreased in the production of the material, resulting in a decreased line speed to increase the duration of the in-line heat treatment. The resulting heat treatment of Example 20 was 15% longer than that of Example 12 and comparable to the heat treatment of Example 6.

Table 7 below shows the LST-ST results from Example 20.

**Table 7:**

| | LST ST [s] |
|---|---|
| Example | 20 |
| | 254.16 |
| | 342.97 |
| | 386.78 |
| | 134.31 |
| | 656.06 |
| Average | 354.86 |
| Std.dev. | 194.08 |
| Min | 134.31 |
| Max | 656.06 |

It can be seen that when increasing the heat treatment time with 15%, it increases the performance in terms of LST ST from 186.30 seconds (12) to 354.86 seconds (20).

### Example 21:

A Spunbond single layer fabric was produced with bicomponent core/sheath configuration, consisting of 70 wt% core and 30 wt% sheath. The core comprised 100% Ziegler-Natta polypropylene. The sheath comprised 67 wt% Ziegler-Natta polypropylene and 33 wt% hydrophobic melt additive (PPM17000 High Load Hydrophobic). The nonwoven was thermally bonded. The single S-layer had a weight of 20 g/m².

### Example 22-24:

Spunbond single layer fabrics were produced with bicomponent core/sheath configuration, consisting of 70 wt% core and 30 wt%. The core comprised 100 wt% Ziegler-Natta polypropylene. The sheath comprised 100-X wt% Ziegler-Natta polypropylene and X wt% hydrophobic melt additive (PPM17000 High Load Hydrophobic). The nonwoven was thermally bonded. The single S-layer had a weight of 20 g/m². After the web making process of the nonwovens they were thermally treated by an in-line IR-heater set to 65% power at the center and 50% at the edge of the nonwoven web.

The contents of the hydrophobic additive in the sheath of the fiber in Examples 22-24 are summarized in below Table 8.

**Table 8:**

| Example | X [wt%] |
|---|---|
| 22 | 10 |
| 23 | 20 |
| 24 | 33 |

Table 9 below shows LST-ST results on Examples 21-24.

**Table 9:**

| Example | 21 | 22 | 23 | 24 |
|---|---|---|---|---|
| | 28.11 | 14.3 | 82.41 | 240.61 |
| | 17.14 | 13.56 | 43.19 | 273.30 |
| | 36.59 | 16.44 | 62.41 | 153.60 |
| | 12.35 | 15.81 | 72.28 | 147.92 |
| | 22.51 | 11.42 | 73.37 | 146.09 |
| | 41.57 | 10.70 | 117.86 | 502.41 |
| | 19.49 | 12.32 | 62.87 | 483.81 |
| | 25.87 | 12.74 | 110.75 | 262.96 |
| | 40.68 | 20.02 | 89.87 | 262.52 |
| | 30.65 | 14.95 | 91.99 | 370.45 |
| | 50.5 | 12.17 | 68.66 | 303.56 |
| | 10.03 | 16.71 | 51.44 | 249.07 |
| | 39.32 | 15.62 | 58.81 | 250.07 |
| | 20.28 | 13.28 | 94.30 | 354.30 |
| | 31.93 | 11.71 | 91.45 | 150.39 |
| Average | 28.47 | 14.12 | 78.11 | 276.74 |
| Std. Dev | 11.73 | 2.50 | 21.32 | 112.54 |
| Min | 10.03 | 10.70 | 43.19 | 146.09 |
| Max | 50.50 | 20.02 | 117.86 | 502.41 |

### Example 25:

A spunbond single layer fabric was produced with bicomponent core/sheath configuration, consisting of 70 wt% core and 30 wt%. The core comprised 100 wt% Ziegler-Natta polypropylene. The sheath comprised 67 wt% propylene-based elastomer (consisting of approx. 15 wt% ethylene) and 33 wt% of a hydrophobic melt additive (PPM17000 High Load Hydrophobic). The nonwoven was thermally bonded. The single S-layer had a weight of 20 g/m².

Table 10 below shows the LST-ST results on Example 25:

**Table 10:**

| Example | 25 |
|---|---|
| | 136.81 |
| | 77.96 |
| | 134.97 |
| | 74.01 |
| | 118.13 |
| | 57.90 |
| | 38.32 |
| | 132.20 |
| | 138.89 |
| | 94.23 |
| Average | 100.34 |
| Std. Dev | 36.86 |
| Min | 38.32 |
| Max | 138.89 |

Example 21 to Example 25 reveals an increase in LST-ST from 28.47 seconds to 100.34 seconds when substituting Ziegler-Natta polypropylene in the sheath of the bicomponent fiber with a propylene-based elastomer in the sheath of the bicomponent fiber.

### Example 26:

A spunbond single layer fabric was produced was produced from 80 wt% Ziegler-Natta polypropylene, 10 wt% of a hydrophobic melt additive (PPM17000 High Load Hydrophobic), and 10 wt% of a Calcium Carbonate masterbatch (Fiberlink 201S). The fabric was thermally bonded. The single S-layer had a weight of 20 g/m². After the web making process of the nonwoven it was thermally treated by an in-line IR-heater set to 65% power at the center and 60% at the edge of the nonwoven web followed by in-line heating in an Omega Drying oven at 120°C.

### Example 27:

A spunbond single layer fabric was produced was produced from 90 wt% Ziegler-Natta polypropylene, and 10 wt% of Calcium Carbonate masterbatch (Fiberlink 201S) and thermally bonded. The single S-layer had a weight of 20 g/m². After the web making process of the nonwoven it was thermally treated in an in-line Omega Drying oven at 120°C.

An overview of Example 26 and 27 is provided in Table 11 below.

**Table 11:**

| | Configuration | | | |
|---|---|---|---|---|
| | S | | | |
| | 20 g/m² | | | |
| Example | PPM17000 in S [%] | Fiberlink 201S [%] | Omega Drying oven temperature [°C] | IR heater, center/edge [%] |
| 26 | 10 | 10 | 120 | 65/60 |
| 27 | 0 | 10 | 120 | N/A |

LST-ST results on Examples 26 and 27 are illustrated in Table 12 below.

**Table 12:**

| | LST-ST [s] | |
|---|---|---|
| Example | 26 | 27 |
| | 604.02 | 4.93 |
| | 530.34 | 5.93 |
| | 898.10 | 5.55 |
| | 685.93 | 5.63 |
| | 838.60 | 6.84 |
| | 522.90 | 4.55 |
| | | 5.45 |
| | | 5.33 |
| | | 5.21 |
| | | 5.69 |
| Average | 679.98 | 5.51 |
| Std.dev. | 158.50 | 0.61 |
| Min | 522.90 | 4.55 |
| Max | 898.10 | 6.84 |

The LST-ST results reveal a LST ST of 5.51 seconds for Example 27, which shows that the presence of CaCO₃ alone does not increase the LST-ST performance. The LST-ST of Example 26 compared to Example 18, reveals that the presence of CaCO₃ and the applied heat treatments of the IR-heater and Omega Drying oven increases the LST ST from 230.33 seconds to 679.98 seconds. When comparing the state of the art of Example 4 to Example 26, the performance increases from 13,59 seconds to 679.98 seconds.

### Examples 28-29:

Two SMMS-multilayered nonwoven fabrics were produced from Ziegler-Natta polypropylene. The fabrics we added a hydrophobic additive (PPM17000 High Load Hydrophobic) to the various layers as described in Table 13. After the web making process of Example 29 the fabric was added a heat treatment with an in-line Omega Drying oven.

Table 13 gives an overview on material layup, additive content and heat treatment.

**Table 13:**

| | Lay-up [g] | | | | Configuration | |
|---|---|---|---|---|---|---|
| | S | M | M | S | SMMS | |
| | 5.5 | 1 | 1 | 5.5 | 13 | |
| Example | PPM17000 per beam [%] | | | | Total PPM17000 [%] | Omega Drying oven temperature [°C] |
| 28 | 0 | 6 | 6 | 6 | 3.5 | N/A |
| 29 | 0 | 6 | 6 | 6 | 3.5 | 120 |

Examples 28-29 were tested for Low Surface Tension Strike Through (LST-ST). The results are summarized in Table 14.

**Table 14:**

| | LST-ST [s] | |
|---|---|---|
| Example | 28 | 29 |
| | 18.35 | 23.99 |
| | 22.44 | 25.67 |
| | 21.70 | 28.36 |
| | 16.52 | 28.99 |
| | 23.13 | 30.71 |
| | 21.09 | 36.43 |
| | 24.01 | 33.29 |
| | 22.42 | 35.08 |
| | 21.30 | 30.98 |
| | 30.27 | 30.78 |
| | 28.86 | 31.71 |
| | 30.13 | 31.09 |
| | 17.95 | 29.27 |
| | 25.50 | 34.52 |
| | 28.48 | 31.24 |
| | 19.34 | 24.87 |
| | 25.11 | 32.50 |
| | 20.44 | 37.55 |
| | 27.07 | 34.13 |
| | 26.68 | 30.61 |
| | 27.65 | 33.90 |
| | 30.11 | 40.04 |
| | 17.50 | 32.49 |
| | 36.19 | 26.82 |
| | 30.73 | 27.60 |
| Average | 24.52 | 31.30 |
| Std.dev. | 5.03 | 4.70 |
| Min | 16.52 | 40.60 |
| Max | 36.19 | 59.50 |

### Examples 30-32:

Three SS materials were produced with the spunbond fibers in both layers being bicomponent fibers of core/sheath configuration with a polyethylene sheath, accounting for 30 wt% of the total fiber, and polypropylene core, accounting for 70 wt% of the total fiber. A hydrophobic additive (PM16310) was added in 17% to the bicomponent's PE sheath of both of S layers for Examples 30-32. After the web making process of Example 31-32 the fabrics were added a heat treatment with an in-line Omega Drying oven of 100°C and 120°C for Example 31 and Example 32, respectively.

Table 15 gives an overview on material layup, additive content and heat treatment.

**Table 15:**

| | Lay-up [g] | | | | Configuration | |
|---|---|---|---|---|---|---|
| | S | | S | | SS | |
| | Core (PP) | Sheath | Core (PP) | Sheath | | |
| | | (PE) | | (PE) | | |
| | 8.75 | 3.75 | 8.75 | 3.75 | 25 | |
| Example | PPM17000 in sheath per beam [%] | | | | Total PPM17000 [%] | Omega Drying oven temperature [°C] |
| 30 | 17 | | 17 | | 5.1 | N/A |
| 31 | 17 | | 17 | | 5.1 | 100 |
| 32 | 17 | | 17 | | 5.1 | 120 |

Examples 30-32 were tested for Low Surface Tension Strike Through (LST-ST). The results are summarized in Table 16.

**Table 16:**

| | LST-ST [s] | | |
|---|---|---|---|
| Example | 30 | 31 | 32 |
| | 21.19 | 16.97 | 16.97 |
| | 33.40 | 22.91 | 22.91 |
| | 12.02 | 18.31 | 18.31 |
| | 22.27 | 22.74 | 22.74 |
| | 12.20 | 22.07 | 22.07 |
| | 24.97 | 28.60 | 37.35 |
| | 23.32 | 15.60 | 20.47 |
| | 26.33 | 44.03 | 33.25 |
| | 24.18 | 26.22 | 33.32 |
| | 16.26 | 20.00 | 37.67 |
| | 26.33 | 23.13 | 37.84 |
| | 20.08 | 29.65 | 29.83 |
| | 41.91 | 20.15 | 24.42 |
| | 47.98 | 20.91 | 43.57 |
| | 15.03 | 17.98 | 26.59 |
| | 13.90 | 17.99 | 42.61 |
| | 34.62 | 17.22 | 27.74 |
| | 25.18 | 12.88 | 22.61 |
| | 12.25 | 16.47 | 30.74 |
| | 32.08 | 18.53 | 35.16 |
| | 34.70 | 26.60 | 31.49 |
| | 13.69 | 21.61 | 43.26 |
| | 45.44 | 34.48 | 54.21 |
| | 21.81 | 14.35 | 35.09 |
| | 36.77 | 32.79 | 40.69 |
| Average | 25.52 | 22.49 | 31.64 |
| Min | 12.02 | 12.88 | 16.97 |
| Max | 47.98 | 44.03 | 54.21 |
| Std. dev. | 10.31 | 7.00 | 9.10 |

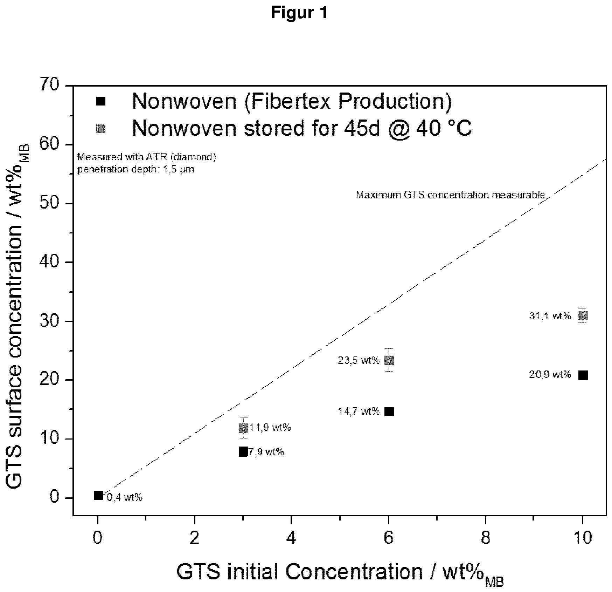

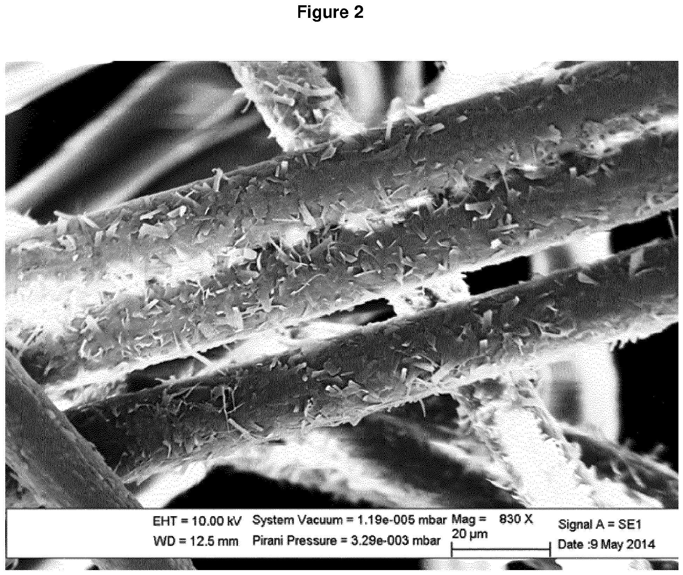

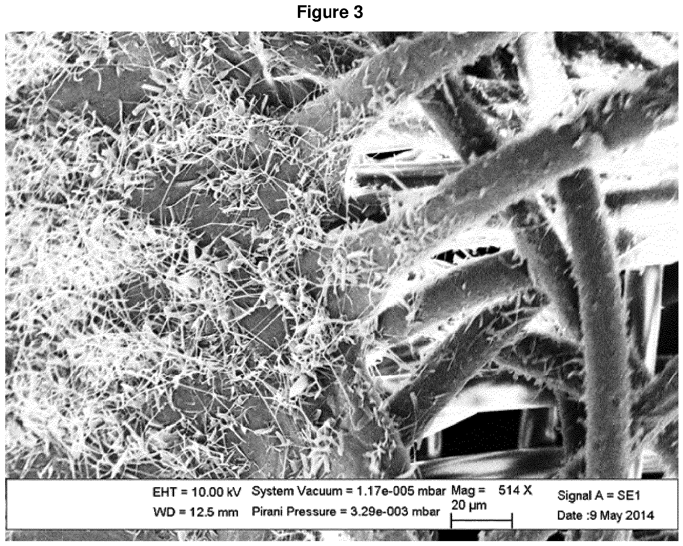

## Claims

1. A method for making a nonwoven fabric comprising forming polymer fibers from a melt of the polymer material and using these fibers to obtain a nonwoven fabric during a subsequent nonwoven fabric formation procedure, wherein the polymer material comprises a thermoplastic polyolefin and wherein the melt of the polymer material comprises a melt additive, which comprises a fatty acid ester derived from C₈₋₂₅, C₁₀₋₂₀ or C₁₄₋₁₈ unsaturated or saturated carboxylic acids,
**characterized in that**
the method comprises thermal bonding at a temperature higher than 40°C below the melting point of the polymer material and, additionally, one or both of the following steps:
a. improving the mobility of the additive by including a filler having a higher thermal conductivity than the polymer material to the polymer material and, optionally, heat-treating the nonwoven in an additional step after it has already been thermally bonded;
b. influencing the polymer crystallinity by including a nucleating agent, branched polymers and/or random co-polymers to the polymer material.

2. The method of claim 1, wherein the polyolefin is polypropylene and/or polyethylene and/or Ethylene-Propylene copolymer or a combination of either of these.

3. The method of any preceding claim, wherein the filler has a thermal conductivity that is higher than the thermal conductivity of the polymer material by at least 0.2 W·m⁻¹·K⁻¹.

4. The method of any preceding claim, wherein the average particle size of the filler is 10 µm or smaller and preferably 1 µm or smaller and/or wherein its thermal conductivity at room temperature is 1 W·m⁻¹·K⁻¹ or more and preferably 2.0 W·m⁻¹·K⁻¹ or more.

5. The method of any preceding claim, wherein the filler comprises CaCO₃ and preferably ground or precipitated CaCO₃.

6. The method of any preceding claim, wherein the nucleating agent comprises a nonitol, a trisamide, a sorbitol-based nucleating agent and/or an inorganic particulate such as, for example, CaCO₃ having a particle size of 10 µm or smaller and preferably 1 µm or smaller.

7. The method of any preceding claim further comprising forming muticomponent polymer fibers from the melt of the one polymer material and a melt of another polymer material, wherein the other polymer material includes less or no additive, wherein at least the majority of the fiber surface is covered by the one polymer material and wherein thermal bonding is carried out at a temperature higher than 40°C below the melting point of the lower melting polymer material.

8. The method of claim 7, wherein the multicomponent fiber is a bicomponent fiber and/or is of core-sheath configuration with the component going back to the one polymer material accounting for the sheath.

9. The method of claim 7 or 8, wherein the one and the other polymer material both are made of or comprise a thermoplastic polymer, preferably a polyolefin such as polypropylene and/or polyethylene and/or Ethylene-Propylene copolymer or a combination of either of these.

10. The method of any preceding claim, wherein the method comprises a continuous process for making a nonwoven fabric, where the fibers are continuously spun and directly dispersed on a carrier belt, preferably comprises making a spunbonded and/or meltblown nonwoven fabric.

11. The method of any preceding claim, wherein the additive comprises a triglyceride.

## Patentansprüche

1. Verfahren zur Herstellung eines Vliesstoffes, umfassend das Bilden von Polymerfasern aus einer Schmelze des Polymermaterials und Verwenden dieser Fasern, um während eines nachfolgenden Vliesstoffbildungsvorgangs einen Vliesstoff zu erhalten, wobei das Polymermaterial ein thermoplastisches Polyolefin aufweist und wobei die Schmelze des Polymermaterials ein Schmelzadditiv aufweist, welches einen Fettsäureester aufweist, der gewonnen wird aus ungesättigten oder gesättigten C₈₋₂₅, C₁₀₋₂₀ oder C₁₄₋₁₈-Carbonsäuren,
**dadurch gekennzeichnet, dass**
das Verfahren die thermische Bindung bei einer Temperatur von mehr als 40 °C unterhalb des Schmelzpunkts des Polymermaterials und zusätzlich einen oder beide der nachfolgenden Schritte umfasst:
a. Verbessern der Mobilität des Additivs durch Einbinden eines Füllstoffs, der eine höhere thermische Leitfähigkeit hat als das Polymermaterial, in das Polymermaterial, und, optional, Wärmebehandeln des Vliesstoffs in einem zusätzlichen Schritt, nachdem es bereits thermisch gebunden wurde;
b. Beeinflussen des Kristallinität des Polymers durch Einbinden eines Keimbildners, verzweigter Polymere und/oder Random Copolymere an das Polymermaterial.

2. Verfahren nach Anspruch 1, wobei das Polyolefin Polypropylen und/oder Polyethylen und/oder Ethylen-Polypropylen-Copolymer oder eine Kombination aus jedem dieser ist.

3. Verfahren nach einem der vorstehenden Ansprüche, wobei der Füllstoff eine thermische Leitfähigkeit hat, die um mindestens 0,2 W•m⁻¹•K⁻¹ höher als die thermische Leitfähigkeit des Polymermaterials ist.

4. Verfahren nach einem der vorstehenden Ansprüche, wobei die mittlere Partikelgröße des Füllstoffs 10 µm oder kleiner und bevorzugt 1 µm oder kleiner ist und/oder wobei seine thermische Leitfähigkeit bei Zimmertemperatur 1 W•m⁻¹•K⁻¹ oder mehr beträgt.

5. Verfahren nach einem der vorstehenden Ansprüche, wobei der Füllstoff CaCO₃ und bevorzugt gemahlenes oder ausgefälltes CaCO₃ aufweist.

6. Verfahren nach einem der vorstehenden Ansprüche, wobei der Keimbildner ein Nonitol, ein Trisamid, einen Keimbildner auf Sorbitol-Basis und/oder ein anorganisches Partikel aufweist, wie beispielsweise CaCO₃ mit einer Partikelgröße von 10 µm oder kleiner und bevorzugt 1 µm oder kleiner.

7. Verfahren nach einem der vorstehenden Ansprüche, ferner umfassend das Bilden von Multikomponentenpolymerfasern aus der Schmelze des einen Polymermaterials und einer Schmelze eines anderen Polymermaterials, wobei das andere Polymermaterial weniger oder kein Additiv aufweist, wobei zumindest die Mehrheit der Faseroberfläche von dem einen Polymermaterial bedeckt ist und wobei thermisches Binden bei einer Temperatur von mehr als 40 °C unterhalb des Schmelzpunkts des niedrigschmelzenderen Polymermaterials durchgeführt wird.

8. Verfahren nach Anspruch 7, wobei die Multikomponentenfaser eine Bikomponentenfaser ist und/oder eine Kern/Mantel-Konfiguration besitzt, bei der die Komponente auf das eine Polymermaterial zurückgeht, das für den Mantel verwendet wird.

9. Verfahren nach Anspruch 7 oder 8, wobei das eine und das andere Polymermaterial beide aus einem thermoplastischen Polymer gebildet sind und ein solches aufweisen, bevorzugt ein Polyolefin wie etwa Polypropylen und/oder Polyethylen und/oder ein Ethylen-Propylen-Copolymer oder eine Kombination aus einem dieser.

10. Verfahren nach einem der vorstehenden Ansprüche, wobei das Verfahren einen Endlosprozess zum Herstellen eines Vliesstoffs umfasst, bei dem die Fasern durchgehend gesponnen und direkt auf ein Trägerband dispergiert werden, bevorzugt das Herstellen eines Spinnvlieses und/oder eines schmelzgeblasenen Vliesstoffs umfasst.

11. Verfahren nach einem der vorstehenden Ansprüche, wobei das Additiv ein Triglycerid aufweist.

## Revendications

1. Procédé destiné à fabriquer un non-tissé comprenant la formation de fibres polymères à partir d'une masse fondue du matériau polymère et l'utilisation de ces fibres pour obtenir un non-tissé au cours d'un processus de formation de non-tissé subséquent, dans lequel le matériau polymère comprend une polyoléfine thermoplastique, et dans lequel la masse fondue du matériau polymère comprend un additif à l'état fondu, lequel comprend un ester d'acide gras dérivé d'acides carboxyliques saturés ou insaturés en C₈₋₂₅, C₁₀₋₂₀ ou C₁₄₋₁₈,
**caractérisé en ce que**
le procédé comprend le liage thermique à une température supérieure à 40 °C au-dessous du point de fusion du matériau polymère et, en outre, une ou deux des étapes suivantes :
a. améliorer la mobilité de l'additif en incluant une charge présentant une conductivité thermique plus élevée que celle du matériau polymère au matériau polymère, et facultativement, traiter thermiquement le non-tissé lors d'une étape supplémentaire alors qu'il a déjà été lié thermiquement, et
b. influencer la cristallinité de polymère en incluant un agent de nucléation, des polymères ramifiés, et/ou des copolymères aléatoires dans le matériau polymère.

2. Procédé selon la revendication 1, dans lequel la polyoléfine est du polypropylène et/ou du polyéthylène, et/ou un copolymère éthylène-propylène, ou une combinaison quelconque entre ceux-ci.

3. Procédé selon l'une quelconque des revendications précédentes, dans lequel la charge présente une conductivité thermique supérieure à la conductivité thermique du matériau polymère à raison d'au moins 0,2 W·m⁻¹·K⁻¹

4. Procédé selon l'une quelconque des revendications précédentes, dans lequel la dimension moyenne des particules de la charge est inférieure ou égale à 10 µm, et de préférence inférieure ou égale à 1 µm, et/ou dans lequel sa conductivité thermique à température ambiante est supérieure ou égale à W·m⁻¹·K⁻¹ et de préférence supérieure ou égale à 2,0 W·m⁻¹·K⁻¹.

5. Procédé selon l'une quelconque des revendications précédentes, dans lequel la charge comprend du CaCO₃, et de préférence du CaCO₃ broyé ou précipité.

6. Procédé selon l'une quelconque des revendications précédentes, dans lequel l'agent de nucléation comprend un nonitol, un trisamide, un agent de nucléation à base de sorbitol, et/ou une particule inorganique telle que par exemple du CaCO₃ présentant une dimension des particules de la charge inférieure ou égale à 10 µm, et de préférence inférieure ou égale à 1 µm.

7. Procédé selon l'une quelconque des revendications précédentes, comprenant en outre la formation de fibres polymères multicomposées à partir de la masse fondue de l'un matériau polymère et d'une masse fondue d'un autre matériau polymère, dans lequel l'autre matériau polymère inclut moins d'additif, ou aucun additif ; dans lequel au moins la majorité de la surface des fibres est recouverte par le un matériau polymère, et dans lequel le liage thermique est réalisé à une température supérieure à 40 °C au-dessous du point de fusion du matériau polymère à point de fusion bas.

8. Procédé selon la revendication 7, dans lequel la fibre multicomposée est une fibre à deux composants, et/ou une configuration de type noyau/gaine, où le composant se réfère à un matériau polymère représentant la gaine.

9. Procédé selon la revendication 7 ou 8, dans lequel l'un et l'autre matériau polymère sont tous deux fabriqués à partir d'un polymère thermoplastique, de préférence d'une polyoléfine telle que du polypropylène et/ou du polyéthylène, et/ou un copolymère éthylène-propylène, ou une combinaison de l'un des ceux-ci.

10. Procédé selon l'une quelconque des revendications précédentes, dans lequel le procédé comprend un processus continu pour fabriquer un non-tissé, où les fibres sont filées en continu, puis directement dispersées sur une courroie transporteuse, et comprend de préférence la réalisation d'un non-tissé obtenu par un procédé filé-lié et/ou de fusion-soufflage.

11. Procédé selon l'une quelconque des revendications précédentes, dans lequel l'additif comprend un triglycéride.
